# EUROPÄISCHE PATENTANMELDUNG

(11) **EP 4 574 110 A1**
(43) Veröffentlichungstag der Anmeldung: **25.06.2025**
(21) Anmeldenummer: 24154920.3
(22) Anmeldetag: 31.01.2024
(51) Int. Cl.: A61F 9/008

(54) **VORRICHTUNG ZUR BEHANDLUNG DER NETZHAUT EINES AUGES**

(30) Priorität: 22.12.2023 DE 102023213307
(71) Anmelder: OD-OS MacuTherm GmbH, 14513 Teltow (DE)
(72) Erfinder: BENGS, Ulrich, 12053 Berlin (DE); MUNDT, Arne, 14476 Potsdam (DE); AMTHOR, Kay-Uwe, 14513 Teltow (DE)
(74) Vertreter: Pfenning, Meinig & Partner mbB

(57) **Zusammenfassung**

Die Erfindung bezieht sich auf eine Vorrichtung zur Behandlung der Netzhaut (1) eines Auges (2)
mit einem Behandlungslaser (5), der einen auf die Netzhaut gerichteten und lenkbaren Behandlungslaserstrahl erzeugt,
mit einer Bildaufnahmeeinrichtung (6) zur Aufnahme von Fundusbildern der Netzhaut und mit einer Positioniereinrichtung (8), die die Bildaufnahmeeinrichtung relativ zur Netzhaut positioniert,
wobei die Positioniereinrichtung eine Nachführeinrichtung aufweist mit:
einer eye-tracking- Einrichtung (8a), die laufend die Ausrichtung des behandelten Auges relativ zur Bildaufnahmeeinrichtung oder relativ zum Behandlungslaser, insbesondere die Blickrichtung des behandelten Auges ermittelt,
einer ersten Verschiebeeinrichtung (8b) zur Verschiebung der Bildaufnahmeeinrichtung in einer oder mehreren Richtungen quer, insbesondere senkrecht, zur Verbindungslinie (10) (zwischen der Bildaufnahmeeinrichtung und der Netzhaut und
einer Nachführungssteuerung (8e), die die erste Verschiebeeinrichtung in Abhängigkeit von der ermittelten Ausrichtung des behandelten Auges und/oder in Abhängigkeit von der Lage des jeweils zu behandelnden Bereiches auf der Netzhaut steuert.

## Beschreibung

Die vorliegende Erfindung liegt auf dem Gebiet der Mechanik, der Elektrotechnik und der Medizintechnik und ist besonders auf dem Gebiet der Augenheilkunde bei einer Laserbehandlung eines Auges einsetzbar.

Eine Vielzahl von Augenkrankheiten und Alterungserscheinungen des menschlichen Auges ist durch verschiedene Arten von Laserbehandlungen behandelbar. Insbesondere Schädigungen und Alterungserscheinungen der Netzhaut des Auges können oft erfolgreich mittels eines Lasers oder durch die Einwirkung einer starken Lichtquelle erfolgreich behandelt werden.

Viele solcher Behandlungen, wie beispielsweise die Behandlung der altersbedingten Macula- Degeneration, erfordern eine genaue Kontrolle der Netzhaut durch bildgebende Verfahren, die die Beurteilung von Bildern der Netzhaut noch während oder kurz nach der Behandlung ermöglichen. Eine solche Beobachtung der Netzhaut ist im zeitlichen Zusammenhang mit der Behandlung meistens schwierig, da im Zuge der Einstrahlung durch den Laser/Lichtstrahl die Pupille des behandelten Auges sich infolge des Pupillenreflexes stark zusammenzieht. Üblicherweise werden die Augen von Patienten vor der Behandlung durch die Gabe von geeigneten Medikamenten, insbesondere Augentropfen, die den Pupillenreflex ausschalten oder vermindern, vorbereitet, so dass das Zusammenziehen der Pupille ausbleibt und die Netzhaut durchgehend gut durch die Pupille zu beobachten ist. Der Nachteil dieses Vorgehens liegt unter anderem darin, dass der jeweilige Patient auch nach der Behandlung noch eine beträchtliche Zeit beeinträchtigt ist und beispielsweise am Straßenverkehr nicht teilnehmen kann. Wird die beschriebene Dilation der Pupille nicht vorgenommen, so wird die Bildgebung der Netzhaut durch die verkleinerte Pupille bereits bei geringer Augenbewegung sehr schwierig.

Der vorliegenden Erfindung liegt daher die Aufgabe zugrunde, eine Vorrichtung zur Behandlung der Netzhaut eines Auges zu schaffen, die die genannten Nachteile vermeidet.

Die Aufgabe wird durch eine Vorrichtung mit den Merkmalen der unabhängigen Patentansprüche gelöst. Die abhängigen Ansprüche stellen vorteilhafte Ausgestaltungen vor.

Die Erfindung bezieht sich demgemäß auf eine Vorrichtung zur Behandlung der Netzhaut eines Auges
mit einem Behandlungslaser, der einen auf die Netzhaut gerichteten und lenkbaren Behandlungslaserstrahl erzeugt,
mit einer Bildaufnahmeeinrichtung zur Aufnahme von Fundusbildern der Netzhaut und mit einer Positioniereinrichtung, die die Bildaufnahmeeinrichtung relativ zur Netzhaut positioniert,
wobei die Positioniereinrichtung eine Nachführeinrichtung aufweist mit: einer eye-tracking- Einrichtung, die laufend die Ausrichtung des behandelten Auges relativ zur Bildaufnahmeeinrichtung oder relativ zum Behandlungslaser, insbesondere die Blickrichtung des behandelten Auges ermittelt,
einer ersten Verschiebeeinrichtung zur Verschiebung der Bildaufnahmeeinrichtung in einer oder mehreren Richtungen quer, insbesondere senkrecht,
zur Verbindungslinie zwischen der Bildaufnahmeeinrichtung und der Netzhaut und einer Nachführungssteuerung, die die erste Verschiebeeinrichtung in Abhängigkeit von der ermittelten Ausrichtung des behandelten Auges und/oder in Abhängigkeit von der Lage des jeweils zu behandelnden Bereiches auf der Netzhaut steuert.

Die beschriebene Lösung ermöglicht es, auch durch eine verengte Pupille während oder im engen zeitlichen Zusammenhang mit einer Laserbehandlung der Netzhaut eines Auges hochqualitative Bilder der Netzhaut mittels der Bildaufnahmeeinrichtung aufzunehmen. Dies gelingt dadurch, dass die Bildaufnahmeeinrichtung durch geeignete Verschiebungen in Echtzeit der Blickrichtung des Auges oder der Ausrichtung der Pupille folgt oder, anders ausgedrückt, vom Auge aus gesehen in der Richtung positioniert wird, die näherungsweise der Verlängerung einer geraden Linie von einer interessierenden Stelle der Netzhaut durch das Zentrum der Pupille entspricht. Wird das Auge bewegt, so kann die Bildaufnahmeeinrichtung jeweils mit minimiertem zeitlichem Verzug nachgeführt werden. Die Verschiebung der Bildaufnahmeeinrichtung kann dazu in einer Richtung oder besser noch in zwei unabhängigen, beispielsweise senkrecht aufeinander stehenden Richtungen erfolgen, die quer zur Blickrichtung des Auges oder quer zu der oben genannten geraden Linie von einer interessierenden Stelle der Netzhaut durch das Zentrum der Pupille orientiert sind. Die Verschiebung kann jeweils entlang einer geraden Linie oder auch entlang einer gekrümmten Linie verlaufen und geführt sein. Beispielsweise kann die Verschiebung der Bildaufnahmeeinrichtung auf einem Bogen, insbesondere auf einem Kreisbogen um das Auge, beispielsweise um das Zentrum der Pupille oder um einen Punkt innerhalb des Auges oder um einen Punkt auf der Netzhaut erfolgen.

Die Bildaufnahmeeinrichtung kann beispielsweise eine Farbbildfunduskamera oder ein Netzhautscanner oder eine andere Art von Kamera, beispielsweise eine Infrarotkamera sein.

Es kann in einer Ausführungsform vorgesehen sein, dass die erste Verschiebeeinrichtung dazu eingerichtet ist, gemeinsam mit der Bildaufnahmeeinrichtung eine Quelle oder ein Einkoppelelement des Behandlungslaserstrahls und/oder eine Beleuchtungseinrichtung für die Netzhaut und/oder eine Einrichtung zur Temperaturmessung an der Netzhaut zu verschieben.

Wenn sich die Blickrichtung des behandelten Auges ändert, so ist bei einer normalen oder sogar verkleinerten Pupillengröße zusätzlich zur Qualität der Bildgebung der Netzhaut zusätzlich sicherzustellen, dass die Netzhaut durch die Pupille gut beleuchtet wird, um ein gutes Bild zu erhalten. Zudem soll auch sichergestellt werden, dass der Laserstrahl des Behandlungslasers die Pupille gut passieren kann. Somit ist es sinnvoll, auch den Laserstrahl des Behandlungslasers und/oder eine Beleuchtungseinrichtung für die Bildaufnahme der Netzhaut durch die Verschiebeeinrichtung mit zu bewegen. Beispielsweise können die Lichtquelle für die Netzhautbeleuchtung und/oder der Behandlungslaser und die Bildaufnahmeeinrichtung in einem beweglichen Modul zusammengefasst sein, das im Ganzen durch die Nachführeinrichtung bewegbar ist. Zu diesem Modul können auch die Linsen für die Strahlführung, beispielsweise eine Kontaktlinse für das Auge oder die dem Auge am nächsten liegende Linse des Strahlführungssystems gehören. Da der Behandlungslaser in vielen Fällen schwer und sperrig ist, kann der Strahl des Behandlungslasers auch in eine Lichtleitfaser eingekoppelt werden, die wenigstens einen beweglichen Abschnitt aufweist. Dieser bewegliche Abschnitt kann durch die Nachführeinrichtung verschiebbar sein und beispielsweise mit der Bildaufnahmeeinrichtung fest verbunden sein. Aus dem beweglichen Abschnitt der Lichtleitfaser kann der Laserstrahl innerhalb des beweglichen Moduls austreten und in die optische Achse der Bildaufnahmeeinrichtung eingekoppelt werden.

Zur Einhaltung von bestimmten Temperaturgrenzen an der Netzhaut während der Bestrahlung durch den Behandlungslaser kann eine Einrichtung zur Messung der Temperatur der Netzhaut am oder in unmittelbarer Nähe des aktuellen Laserspots auf der Netzhaut vorgesehen sein. Durch eine solche Temperaturmesseinrichtung kann eine Steuerung der Laserleistung oder anderer Laserparameter auf der Grundlage der Temperaturmessung realisiert werden.

Mit Hilfe der Temperaturmessung kann ein geschlossener Regelkreis zur Regelung der Netzhauttemperatur während der Behandlung eingerichtet werden.

Die Vorrichtung umfasst für diesen Zweck einen Gewebetemperaturregelmechanismus mit einer Gewebetemperaturmessvorrichtung die Temperaturmesswerte des Netzhautgewebes an eine Steuerungseinrichtung des Behandlungslasers sendet. Auf diese Weise kann der Regelkreis aufgebaut werden, der dafür sorgt, dass die Zieltemperaturen erreicht und eingehalten werden können. Die Temperaturmesseinrichtung basiert in einer Ausführungsform auf der "Grüneisen"-Formel und misst die Absorption kurzer Strahlungsimpulse, die von einem Anregungslaser auf die Netzhaut gesendet werden. Die unter anderem von der Temperatur der Netzhaut abhängige Absorptionsintensität wird durch Antwortsignale in Form von Druckwellen erfasst, die durch den abrupten Temperaturanstieg auf der Netzhaut erzeugt werden, der durch die Strahlungsimpulse des Anregungslasers hervorgerufen wird. Die Druckwellen werden von einem Druckwandler, beispielsweise einem Piezowandler, aufgefangen, der beispielsweise auf das Auge aufgesetzt werden kann. Die von dem Druckwandler erzeugten elektrischen Signale werden in Temperaturwerte umgewandelt.

Bei dem Anregungslaser kann es sich um einen vom Behandlungslaser getrennten Laser handeln, in einigen Ausführungsformen kann aber auch der Behandlungslaser selbst als Anregungslaser für die zur Temperaturmessung ausgesandten Strahlungsimpulse verwendet werden. Der Anregungslaser kann durch die Nachführeinrichtung, insbesondere durch die erste Verschiebeeinrichtung, gemeinsam mit der Bildaufnahmeeinrichtung verschoben werden, um auch bei einer Bewegung des Auges eine kontinuierliche Temperaturerfassung zu gewährleisten.

Die Temperaturmesseinrichtung kann dazu verwendet werden, einen geschlossenen Regelkreis mit der Prozesssteuerungseinrichtung aufzubauen und die Temperatur an einem aktuellen Laserspot zuverlässig auf die Zieltemperatur zu begrenzen. Dazu kann die Prozesssteuerungseinrichtung anhand der gemessenen Temperatur die Laserleistung, das Tastverhältnis, den Laserspotdurchmesser oder auch die Wellenlänge des Behandlungslasers steuern.

Zur Temperaturmessung kann auch anstelle des oben beschriebenen Verfahrens beispielsweise eine Messung unter Verwendung optischer Kohärenztomographie durchgeführt werden, wobei die Messeinrichtung ebenfalls mittels der Nachführeinrichtung bewegbar sein kann. Eine Strahlenführung für die Aufnahme der Netzhaut kann entlang der optischen Achse des Behandlungslasers verlaufen und sich auch derselben optischen Strahlführungselemente bedienen wie der Behandlungslaser.

Es kann weiter in einer Ausführungsform vorgesehen sein, dass die eye-tracking- Einrichtung dazu eingerichtet ist, die Ausrichtung des Auges, dessen Netzhaut behandelt wird, durch Erfassung der Position und/oder Bewegung von einem oder mehreren Elementen des Auges auf seiner der Bildaufnahmeeinrichtung zugewandten Seite zu ermitteln.

Die Eye-tracking-Einrichtung kann beispielsweise Bilder von der Vorderseite des Auges aufnehmen, die dem Behandlungslaser und der Bildaufnahmeeinrichtung zugewandt ist. Weiter kann mittels eines reflektierten Lichtstrahls die Augenoberfläche und ihre Bewegung verfolgt werden, indem der Lichtreflex verfolgt wird. Es kann auch die Bewegung einer Oberflächenstruktur eines Teils des Auges durch ein bildgebendes Verfahren verfolgt werden.

Außerdem kann vorgesehen sein, dass die eye-tracking- Einrichtung eine oder zwei oder mehr als zwei Kameras aufweist, die ein oder mehrere Elemente auf der der Bildaufnahmeeinrichtung zugewandten Seite des behandelten Auges erfassen.

Eine oder mehrere Kameras können beispielsweise die Iris oder einen Teil der Iris oder die Pupille des Auges erfassen und verfolgen und somit die Augenbewegungen erfassen.

Bei einer Erfassung durch mehrere voneinander beabstandete Kameras kann die Bewegung des Auges auch in drei Dimensionen erfasst werden. Damit stehen Informationen über den Abstand zwischen dem Auge und der Bildaufnahmeeinrichtung zur Verfügung, die für die Positionierung der Bildaufnahmeeinrichtung verwendet werden können.

Es kann weiter in einer Ausführungsform vorgesehen sein, dass die Nachführeinrichtung eine zweite Verschiebeeinrichtung zur Verschiebung der Bildaufnahmeeinrichtung in Richtung der Verbindungslinie zwischen der Bildaufnahmeeinrichtung und der Netzhaut oder der Pupille aufweist.

Es kann, insbesondere bei einer Verfolgung des Auges in drei Dimensionen, der Abstand des Auges von der Bildaufnahmeeinrichtung erfasst werden und dann dieser Abstand mittels der zweiten Verschiebeeinrichtung auf einen Sollzustand gebracht werden, in dem ein scharfes Bild der Netzhaut aufgenommen wird. Das Bild, das die Bildaufnahmeeinrichtung von der Netzhaut aufnimmt, ist in vielen Fällen mit einer geringen Tiefenschärfe versehen, das heißt, es ist nur in einem sehr begrenzten Entfernungsintervall scharf. Daher ist es vorteilhaft, wenn die Entfernung zwischen der Netzhaut und dem Sensor oder einer Bildebene der Bildaufnahmeeinrichtung laufend auf einem Sollwert gehalten werden kann.

Es kann weiter in einer Ausführungsform vorgesehen sein, dass das mit der Bildaufnahmeeinrichtung erfasste Bild der Netzhaut bei der Erfassung der Ausrichtung des Auges berücksichtigt wird. Die Berücksichtigung des Netzhautbildes erlaubt gegenüber der Auswertung der Messergebnisse der eye-tracking- Einrichtung, die die Augenoberfläche oder die Element an der der Bildaufnahmeeinrichtung zugewandten Seite des Auges erfasst, eine genauere Messung, wobei allerdings der Erfassungsbereich begrenzt ist, so dass es oft sinnvoll ist, zunächst die Ergebnisse der eye-tracking- Einrichtung auszuwerten und darauf eine nachfolgende Feinpositionierung mit den Bildern der Bildaufnahmeeinrichtung vorzunehmen.

Eine Ausführungsform der Vorrichtung kann weiter vorsehen, dass die Nachführeinrichtung eine Winkelnachführeinrichtung aufweist, die bei einer Verschiebung der Bildaufnahmeeinrichtung diese in Abhängigkeit von Richtung und Maß der Verschiebung durch eine Dreh- oder Schwenkbewegung auf die Netzhaut des behandelten Auges ausrichtet.

Wird die Bildaufnahmeeinrichtung gegenüber dem Auge verschoben, so ist insbesondere in dem Fall, dass die Bildaufnahmeeinrichtung auf einer geraden

Linie quer zur Verbindungslinie zwischen der Einrichtung und dem Auge verschoben wird, damit zu rechnen, dass die Bildaufnahmeeinrichtung nach der Verschiebung nicht mehr optimal auf das Auge gerichtet ist. Eine Korrektur der Ausrichtung durch eine Winkelnachführeinrichtung ist somit vorteilhaft. Es kann auch vorgesehen sein, dass die Nachführeinrichtung eine Zuordnungseinrichtung aufweist, die verschiedenen Ausrichtungen des Auges Schwenk- oder Drehrichtungen und/oder Verschiebungsrichtungen der Nachführeinrichtung und Schwenk- oder Drehwinkel und/oder Verschiebungsstrecken zuordnet, um die die Bildaufnahmeeinrichtung durch die Winkelnachführeinrichtung zu schwenken oder drehen und/oder durch eine erste oder zweite Verschiebeeinrichtung zu verschieben ist.

Es kann weiter in einer Ausführungsform vorgesehen sein, dass die Nachführeinrichtung eine Zuordnungseinrichtung aufweist, die verschiedenen Ausrichtungen des Auges Zielpositionen der Bildaufnahmeeinrichtung zuordnet, auf die diese ausgerichtet werden soll. Die jeweiligen Zielpositionen können von der Nachführeinrichtung zur Nachführungssteuerung verwendet werden Eine Ausführungsform kann auch vorsehen, dass die Nachführeinrichtung eine Zuordnungseinrichtung aufweist, die verschiedenen Ausrichtungen des Auges Verschiebungsrichtungen und Verschiebungsstrecken zuordnet, um die die Bildaufnahmeeinrichtung zu verschieben ist.

Bei einer Augenbewegung dreht sich das Auge näherungsweise um einen Punkt, der an einer Stelle im Augeninneren liegt. Die Bewegung der Netzhaut bei einer Augenbewegung ist jedoch nur schwierig genau zu erfassen, da sie unter anderem von der Anlenkung der verschiedenen Augenmuskeln an den Augapfel abhängt. Für eine optimierte Nachführung der Bildaufnahmeeinrichtung ist somit eine Zuordnung (ein mapping) von Augenbewegungen oder Ausrichtungen des Auges zu den entsprechenden Bewegungen oder Positionen der Netzhaut hilfreich. Diese Zuordnung kann durch eine selbstlernende Einrichtung gelernt werden oder es kann durch eine Versuchsreihe eine Zuordnungstabelle oder ein Zuordnungsalgorithmus erstellt und programmiert werden.

Es kann auch zudem eine Leiteinrichtung für die Blickrichtung des Auges, dessen Netzhaut behandelt wird, vorgesehen sein, wobei durch die Leiteinrichtung Bilder von wenigstens einem, insbesondere wenigstens zwei, weiter insbesondere fünf Graphikelementen mittels separat und im Abstand voneinander durch die Pupille geführter Lichtstrahlen auf der Netzhaut erzeugt werden und wobei in Abhängigkeit von der Blickrichtung des behandelten Auges von den Lichtstrahlen und den durch diese erzeugten Bildern von Graphikelementen einer/eines oder mehrere durch eine elektronische Steuerung oder optisch durch die Iris des behandelten Auges blockiert werden können.

Eine solche Leiteinrichtung soll der Person, deren Auge behandelt wird, signalisieren, wenn das Auge sich bewegt oder nicht optimal ausgerichtet ist. Beispielsweise können an den Rändern des Sichtfeldes des Auges Graphikelemente für die Person sichtbar sein, die durch eine nicht erwünschte Bewegung des Auges aus dem Sichtfeld verschwinden. Das Verschwinden eines Graphikelementes aus dem Sichtfeld kann einerseits rein optisch dadurch geschehen, dass der Rand der Iris nach der Augenbewegung das Graphikelement blockiert, indem die Lichtstrahlen, die die Abbildung dieses Graphikelementes auf der Netzhaut bewirken, durch die Fläche der Iris ausgeblendet werden.

Es kann aber auch eine elektronische Steuerung vorgesehen sein, die aufgrund einer Detektion der Augenausrichtung, beispielsweise durch die Eye-tracking- Einrichtung, die Erzeugung bestimmter Graphikelemente bei einer Dezentrierung der Blickrichtung oder der Ausrichtung des Auges stoppt oder andere Graphikelemente erzeugt.

Die Leiteinrichtung kann auch wenigstens ein zentrales Graphikelement durch einen Lichtstrahl erzeugen, der durch die Mitte der Pupille fällt, so dass dieses zentrale Graphikelement dauerhaft wahrnehmbar ist und dem Patienten eine Orientierung bietet, wohin er seinen Blick richten soll.

Die jeweils verbleibenden oder neu erzeugten, durch die Person bei einer Fehlausrichtung des Auges erkennbaren Graphikelemente können eine Bedeutung tragen, die von der Person erkennbar ist und die sie dazu anregt, das Auge in einer gewünschten Richtung neu auszurichten oder den Blick in eine bestimmte gewünschte Richtung zu lenken.

Es kann zudem vorgesehen sein, dass der Behandlungslaser für eine Behandlung der Netzhaut mit einer Bestrahlungsintensität eingerichtet ist, die das Gewebe der Netzhaut für einen kontrollierbaren Zeitraum auf Temperaturen von weniger als 58 Grad Celsius, insbesondere mit einer Höchsttemperatur zwischen 48 Grad und 58 Grad, weiter insbesondere mit einer Höchsttemperatur zwischen 50 Grad und 55 Grad erwärmt.

Gemäß den geltenden medizinischen Behandlungsrichtlinien sollen bisher Netzhauterkrankungen im frühen Stadium nicht durch eine Laserbehandlung therapiert werden, da diese in den bisher bekannten Therapieformen Gewebe zerstört oder zumindest schädigt.

Die erfindungsgemäße Vorrichtung zur Netzhautbehandlung kann jedoch beispielsweise ausschließlich oder zusätzlich zu gewebeverändernden weiteren Behandlungsoptionen eine Behandlungsoption ermöglichen, bei der die Temperaturen, die mit ihr erreicht werden, oberhalb der für den Laserbetrieb zulässigen Augensicherheitsschwelle des Laserbetriebs gewählt werden können, jedoch unterhalb von Temperaturen, bei denen unmittelbar oder verzögert eine Gewebeveränderung durch den Laser hervorgerufen wird. Es hat sich herausgestellt, dass durch die Erwärmung bei einer derart temperaturbegrenzt gestalteten Behandlung physiologische Prozesse im Gewebe in Gang gesetzt oder beschleunigt werden, die mittelbar einen positiven Effekt auf die Gesundheit und Funktionsweise des Zielgewebes haben, ohne dieses unmittelbar zu verändern. Beispielsweise werden Enzyme, Hormone und Botenstoffe ausgeschüttet oder vermehrt ausgeschüttet, die die Gewebefunktionalität verbessern und damit den Fortschritt von Netzhauterkrankungen verhindern oder verlangsamen. Damit kann eine intravitreale Injektion von Medikamenten oder eine andere aufwändige Intervention vermieden werden. Eine derartige Behandlung kann in vielen Fällen für die Netzhaut oder Teile der Netzhaut automatisiert durchgeführt werden.

Die Erreichung der gewünschten Temperaturen der Netzhaut kann durch Erfahrungswerte der Bestrahlungsparameter eingestellt werden, die beispielsweise auch durch ein selbstlernendes System in der Steuerungseinrichtung des Behandlungslasers ermittelt werden können. Es kann jedoch auch eine Regelung der Behandlungsintensität mittels einer nichtinvasiven Echtzeitmessung und Überwachung der Temperatur am jeweiligen Bestrahlungsort durch ein spektroskopisches Verfahren vorgesehen sein, wie es beispielsweise weiter oben bereits angesprochen und in der Offenlegungsschrift EP1279385A1 beschrieben ist. Dabei wird eine Reaktion des Gewebes auf einen kurzen Strahlungsimpuls in Form von transienten Drucksignalen einer Druckwelle beobachtet, wobei die Signale über ihre Abhängigkeit von dem sogenannten Grüneisenkoeffizienten eine Temperaturbestimmung des Gewebes erlauben. Die Zielgröße ist dabei eine zu erreichende Zieltemperatur des Gewebes und die Stellgröße ist die steuerbare Strahlungsintensität der Lichtquelle oder die Bestrahlungsdauer oder ein Bestrahlungsmodus, der eine Modulation der Lichtquelle oder weitere Größen umfassen kann.

In einer Ausführungsform kann vorgesehen sein, dass die Behandlungsvorrichtung dazu eingerichtet ist, die Netzhaut Spot für Spot nacheinander jeweils durch Bestrahlung mit dem Laser für einen Zeitraum von weniger als 500 msec, insbesondere weniger als 200 msec auf eine Temperatur zwischen 50 und 55 Grad Celsius zu erwärmen.

Weiter kann vorgesehen sein, dass der Zeitraum der Bestrahlung mit der genannten Leistung wenigstens 10 msec (Millisekunden), weiter insbesondere wenigstens 50 msec, weiter insbesondere wenigstens 100 oder 150 msec beträgt.

Beispielsweise können sich somit Zeiträume zwischen 50 und 200 msec oder zwischen 50 und 500 msec oder zwischen 100 und 200 msec oder zwischen 100 und 500 msec oder zwischen 150 und 500 msec ergeben.

Durch Einhaltung eines optimalen Zeitfensters kann einerseits sichergestellt werden, dass eine Zieltemperatur des Gewebes der Netzhaut oder in ihrer unmittelbaren Umgebung in dem Temperaturfenster erreicht wird und dass andererseits kritische Grenztemperaturen nicht überschritten werden, so dass Gewebeschädigungen sicher vermieden werden können.

Die Behandlungsvorrichtung kann auch dazu eingerichtet sein, jeweils auf den zu behandelnden Flächen der Netzhaut Spot für Spot nacheinander jeweils durch Bestrahlung mit dem Laser für einen Zeitraum von weniger als 500 msec (Millisekunden), insbesondere weniger als 200 msec mit einer Leistungsdichte zwischen 150 W/cm2 und 350 W/cm2, insbesondere zwischen 180 W/cm2 und 300 W/cm2 zu bestrahlen.

Auch in diesem Fall können vorteilhaft die weiter oben genannten Grenzen für die Bestrahlungszeit mit den genannten Leistungen gelten sowie die oben angegebenen Zeitfenster.

Dier Erfindung bezieht sich außer auf eine Vorrichtung der oben beschriebenen Art auch auf ein Verfahren zur Behandlung der Netzhaut eines Auges mit einem Behandlungslaser, der einen auf die Netzhaut gerichteten und lenkbaren Behandlungslaserstrahl erzeugt, wobei während der Laserbehandlung mit einer Bildaufnahmeeinrichtung Fundusbilder der Netzhaut aufgenommen werden,
und wobei während der Laserbehandlung der Netzhaut mit einer eye-tracking- Einrichtung laufend die Ausrichtung, insbesondere die Blickrichtung des behandelten Auges ermittelt wird und
mit einer Nachführungssteuerung in Abhängigkeit von der ermittelten Ausrichtung des behandelten Auges eine erste Verschiebeeinrichtung zur Verschiebung der Bildaufnahmeeinrichtung in einer oder mehreren Richtungen quer, insbesondere senkrecht, zur Verbindungslinie zwischen der Bildaufnahmeeinrichtung und der Netzhaut gesteuert wird.

Im Rahmen des Verfahrens kann auch die zweite Verschiebeeinrichtung und die Winkelnachführeinrichtung gesteuert werden und außer der eye-tracking-Einrichtung, die sich beispielsweise einer oder mehrerer gesonderter Kameras bedienen kann, kann auch das durch die Bildaufnahmeeinrichtung aufgenommene Bild der Netzhaut zur Ermittlung der Orientierung des Auges herangezogen werden.

Im Folgenden wird die Erfindung anhand von Ausführungsbeispielen in Figuren einer Zeichnung gezeigt und nachfolgend beschrieben.

Dabei zeigen
- Figur 1:: eine schematische, teilweise perspektivische Darstellung der Vorrichtung mit einer Verschiebeeinrichtung,
- Figur 2:: eine schematische Darstellung der Vorrichtung mit einer Winkelnachführeinrichtung,
- Figur 3:: eine Darstellung der Bildaufnahmeeinrichtung, einer Beleuchtungseinrichtung und einem Behandlungslaser, die in einer Einheit zusammengefasst sind,
- Figur 4:: eine Darstellung eines Funktionsschemas der Erfindung,
- Figur 5:: eine Darstellung eines Ablaufschemas,
- Figuren 6, 7:: beispielhafte Graphikelemente einer Leiteinrichtung,
- Figuren 8, 9:: ein Anwendungsbeispiel der Leiteinrichtung.

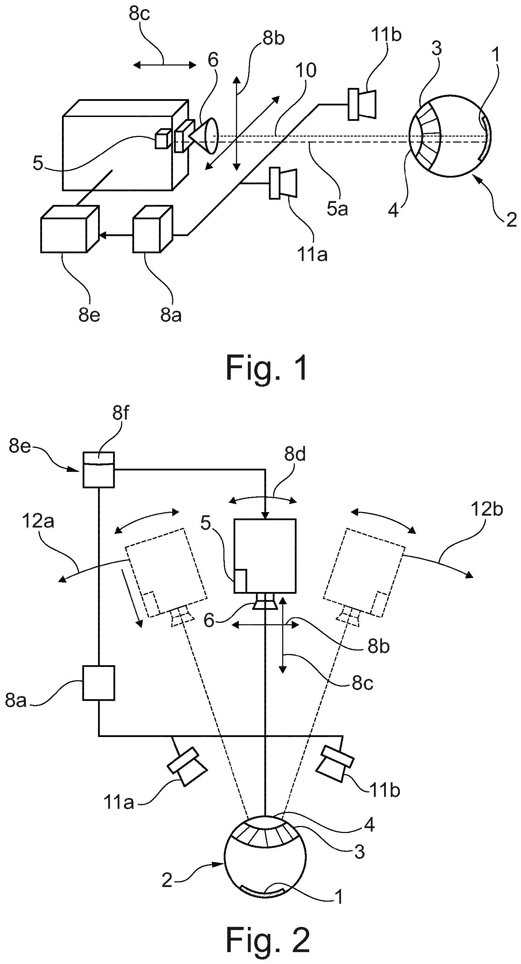

Die Figur 1 zeigt in einer teilweise perspektivischen Darstellung eine Bildaufnahmeeinrichtung 6 in Form einer Farbbildfunduskamera, die entlang der Verbindungslinie 10 zwischen der Bildaufnahmeeinrichtung und dem Auge 2 ein Bild der Netzhaut 1 auf der Rückseite des Auges aufnimmt. Mit der Bildaufnahmeeinrichtung ist eine Quelle 5 für einen Behandlungslaserstrahl 5a verbunden, so dass sich ein gemeinsames Modul, das die Bildaufnahmeeinrichtung und die Laserstrahlquelle enthält, gemeinsam bewegen lässt. Wie weiter unten noch dargestellt wird, kann in diesem Modul auch eine in der Figur 1 nicht dargestellte Beleuchtungseinrichtung mit enthalten sein. Es sind weiter zwei voneinander beabstandete Kameras 11a, 11b dargestellt, die miteinander gekoppelt und auf die Vorderseite des Auges 2 gerichtet sind und die Augenbewegungen in drei Dimensionen erfassen. Die Bilder der Kameras werden in einer Verarbeitungseinrichtung 8a zu einer Information über die Position und Ausrichtung des Auges vorverarbeitet und diese Information wird einer Nachführungssteuerung 8e übermittelt. Die Nachführungssteuerung steuert eine erste Verschiebeeinrichtung, deren Funktion und Verschieberichtungen durch die beiden senkrecht aufeinander stehenden Pfeile 8b symbolisch repräsentiert sind. Die erste Verschiebeeinrichtung weist einen oder mehrere Antriebseinrichtungen, beispielsweise in der Form von Elektromotoren oder Piezoantrieben auf, die die Bildaufnahmeeinrichtung in zwei Richtungen quer oder senkrecht zu der Verbindungslinie 10 oder dem Laserstrahl 5a verschieben.

Wichtig ist dabei, dass die Verschiebungsrichtung oder die Verschiebungsrichtungen zumindest eine Komponente aufweisen, die senkrecht zu der Verbindungslinie 10 verläuft.

Der Laserstrahl 5a verläuft parallel zu der Verbindungslinie 10 oder genau auf dieser Linie und die optische Achse der Bildaufnahmeeinrichtung ist identisch mit der optischen Achse des Laserstrahls 5a oder zumindest parallel zu dieser. Beide optischen Achsen passieren dieselben optischen Elemente, beispielweise Fokussierungs- und Kollimationslinsen. Typischerweise werden die der Bildaufnahmeeinrichtung übermittelten Lichtstrahlen und der Laserstrahl und optional auch ein Beleuchtungsstrahl mittels halbdurchlässiger Spiegel auf dieselbe Achse gelenkt. Auf dieser Achse kann auch optional eine Temperaturmessung entweder durch eine pyrometrische Aufnahme oder eine thermooptische Messung mit einer Impulsanregung durch einen Laser und eine Aufnahme einer durch Laserstrahlabsorption erzeugten Druckwelle stattfinden. Als Anregungslaser kann dabei beispielsweise auch der Behandlungslaser dienen. Die Druckwelle kann dann direkt durch einen auf das Auge aufgesetzten, nicht dargestellten Sensor erfolgen. Mittels der Temperaturmessung während der Laserbehandlung kann beispielsweise die Intensität des Behandlungslasers durch geeignete Parameter wie den duty cycle oder die Größe des Laserspots geregelt werden, um, falls dies gewünscht ist, die Temperatur der Netzhaut in einem bestimmten Fenster zu halten.

Wie weiter unten gezeigt wird, muss der Laser 5 nicht unbedingt direkt mit der Bildaufnahmeeinrichtung verbunden sein, sondern der Laserstrahl kann auch mittels einer beweglichen Lichtleitfaser zu dem Modul geleitet werden, das die Bildaufnahmeeinrichtung enthält. Als Strahlquelle des Laserstrahls kann dann die Einkoppelstelle betrachtet werden, an der das Licht aus der Austrittsfläche der Lichtleitfaser austritt und in die optischen Elemente entlang der optischen Achse eingestrahlt wird.

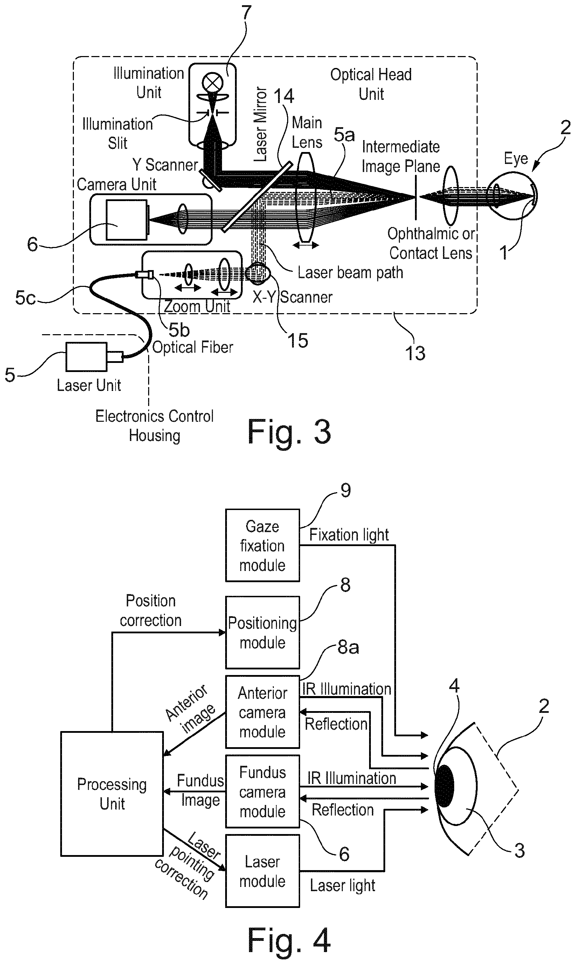

Mit dem Pfeil 8c ist symbolisch eine zweite Verschiebeeinrichtung dargestellt, die einen Antrieb aufweist, der die Bildaufnahmeeinrichtung auf der Verbindungslinie 10 verschiebt, um zu erreichen, dass die Austrittspupille des optischen Systems und Augenpupille überlagert werden Ein weiteres Ziel besteht darin, eine Fokussierung auf die Netzhaut 1 zu erreichen und diese auch jeweils beizubehalten, wenn das Auge sich bewegt. Zur Fokussierung kann beispielsweise die "Main Lens" oder "Ophthalmie Lens" aus Fig. 3 in Richtung der Verbindungslinie 10 verschoben werden. Zu diesem Zweck erlaubt die Eye-tracking- Einrichtung neben der Ermittlung der Ausrichtung des Auges 2 auch die Ermittlung der dreidimensionalen Position des Auges. Allerdings kann noch eine Zuordnungseinrichtung hilfreich sein, die den verschiedenen Positionen und/oder Ausrichtungen des Auges, die durch die Eye-tracking- Einrichtung aufgrund äußerer Merkmale und Orientierungselemente des Auges ermittelt werden, jeweils Bewegungen und Positionen der Netzhaut oder spezieller Punkte der Netzhaut zuzuordnen, da das Auge bei seine Bewegung üblicherweise nicht nur um eine oder mehrere feste Achsen rotiert, sondern aufgrund der Muskelanbindung einer Vielzahl von Muskeln eine kompliziertere Bewegung ausführt.

Die Figur 2 zeigt schematisch, dass die erste Verschiebeeinrichtung außer einer geraden Verschiebung der Bildaufnahmeeinrichtung 6 diese auch entlang einer bogenförmigen, beispielsweise kreisbogenförmigen Linie verschieben kann, was durch die Pfeile 12a, 12b angedeutet ist. Eine solche bogenförmige Verschiebung kann beispielsweise durch eine Kulissenführung oder mehrere unabhängige, aufeinander abgestimmte Antriebseinheiten für verschiedene Antriebsrichtungen realisiert sein.

Gleichzeitig und zusätzlich kann die Bildaufnahmeeinrichtung auch mechanisch auf einer Schiene oder Kulisse oder durch eine Winkelnachführeinrichtung 8d mit einem eigenen Antrieb gedreht oder geschwenkt werden, um bei einer Verschiebung die Ausrichtung auf das Auge 2 oder auf einen festen Punkt auf der Netzhaut beizubehalten. Gemeinsam mit der Bildaufnahmeeinrichtung kann auch der Behandlungslaser und eine optionale Temperaturmesseinrichtung zur Messung der Temperatur der Netzhaut im Bereich des Laserspots verschoben und/oder geschwenkt werden.

Die Figur 3 zeigt ein insgesamt bewegliches, zusammenhängendes festes Modul 13, das eine Bildaufnahmeeinrichtung 6, eine Beleuchtungseinrichtung 7 sowie eine Einkoppelstelle 5b aufweist, an der der Laserstrahl des Behandlungslasers aus einer beweglichen Lichtleitfaser 5c austritt. Der Laserstrahl 5b wird mittels eines Spiegels 14 in den optischen Pfad eingespiegelt, der auch der Bildachse der Bildaufnahmeeinrichtung und der Beleuchtungseinrichtung 7 entspricht. Mittels des MEMS- Scanners 15 kann der Laserstrahl des Behandlungslasers auf beliebige gewünschte Stellen der Netzhaut 1 des Auges 2 gelenkt werden. Dieser Laserstrahl kann neben der eigentlichen Netzhautbehandlung auch mit kurzen Impulsen zur Temperaturmessung genutzt werden.

Die Figur 4 zeigt verschiedene Funktionseinheiten der Vorrichtung, wie beispielsweise die Positioniereinrichtung 8, die Kameraeinheit der eye-tracking-Einrichtung, die Bildaufnahmeeinrichtung 6, das Lasermodul 5 und eine Leiteinrichtung 9, die weiter unten noch näher beschrieben ist.

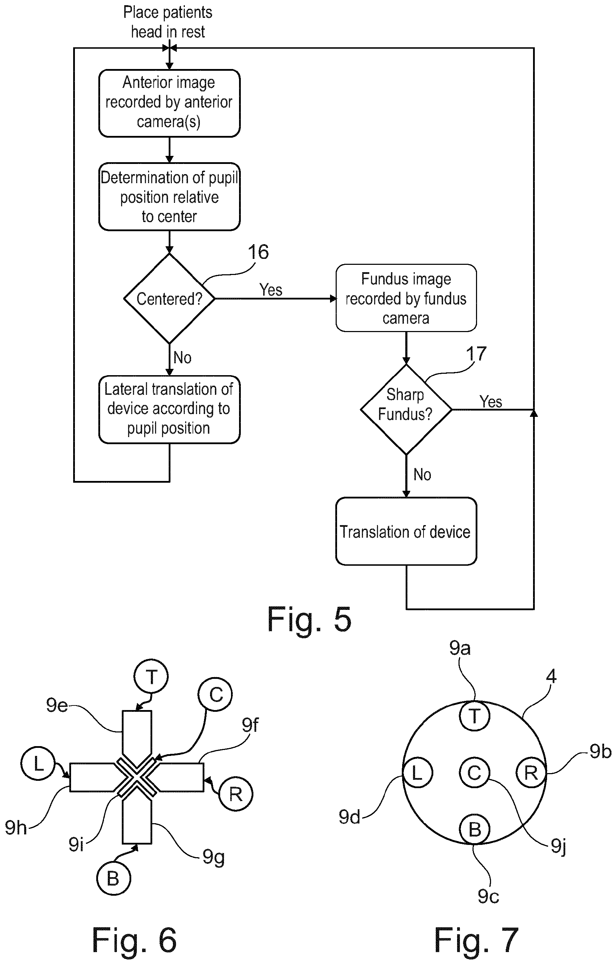

Die Figur 5 zeigt ein Ablaufdiagramm mit Informationserfassungsschritten und Entscheidungsschritten 16, 17. Im Entscheidungsschritt 16 wird, falls die Position der Bildaufnahmeeinrichtung nicht zur Ausrichtung der Pupille des Auges passt, die erste Verschiebeeinrichtung 8b aktiviert.

Im Entscheidungsschritt 17 wird, falls der Abstand der Bildaufnahmeeinrichtung zur Netzhaut des Auges nicht passend für eine scharfe Abbildung ist, die zweite Verschiebeeinrichtung 8c aktiviert.

Die Figur 6 zeigt fünf Graphikelemente 9e, 9f, 9g, 9h, 9i, von denen die vier Elemente 9e, 9f, 9g, 9h die Form von Pfeilen haben und die durch eine nicht dargestellte Projektionseinrichtung oder Bilderzeugungseinrichtung auf die Netzhaut 1 abgebildet werden. Das Graphikelement 9i hat die Form eines Kreuzes und dient der Angabe des zentralen Bereichs, auf den der Patient seinen Blick richten soll. Ist das Auge gerade ausgerichtet, so nimmt es alle fünf Graphikelemente wahr. Bewegt sich das Auge, so wird, da die Pupille ohne eine Medikamentengabe relativ klein ist, wenigstens eines der Graphikelemente/einer der Pfeile 9e, 9f, 9g, 9h durch den Rand der Iris, der sich mit dem Auge bewegt, blockiert. Blickt beispielsweise das Auge nach rechts, so wird als erstes das Graphikelement 9h auf der linken Seite ausgeblendet, so dass das Graphikelement 9f auf der rechten Seite mehr Aufmerksamkeit erfährt. Dieses stellt einen Pfeil dar, der die gewünschte Änderung der Blickrichtung nach links repräsentiert, so dass die Person, die behandelt wird, die Aufforderung wahrnimmt, das Auge zur Korrektur der Ausrichtung wieder nach links auf das Kreuz 9i zu richten. Dasselbe Prinzip gilt für eine Abweichung der Ausrichtung des Auges nach oben und unten. Neben den Graphikelementen sind in der Figur 6 zusätzlich zu den Bezugszeichen 9e, 9f, 9g, 9h, 9i noch die Bezeichnungen T, R, B, L, C eingetragen die für die englischen Bezeichnungen der jeweiligen Lichtstrahlen stehen, die jeweils eines der Graphikelemente erzeugen, nämlich Top, Right, Bottom, Left und Center.

Diese Lichtstrahlen sind in der Figur 7 innerhalb eines Kreises eingezeichnet, der den äußeren Rand der Pupille 4 oder den inneren Rand der Iris bezeichnet. Bei der in der Figur 7 dargestellten Situation, in der die Blickrichtung des Auges zentrisch ausgerichtet ist, können alle fünf Lichtstrahlen T, R, B, L, C, bezeichnet mit den Bezugszeichen 9a, 9b, 9c, 9d, 9j ungehindert durch die Pupille hindurchtreten, das heißt die fünf in der Figur 6 dargestellten Graphikelemente 9e, 9f, 9g, 9h, 9i werden alle wahrgenommen.

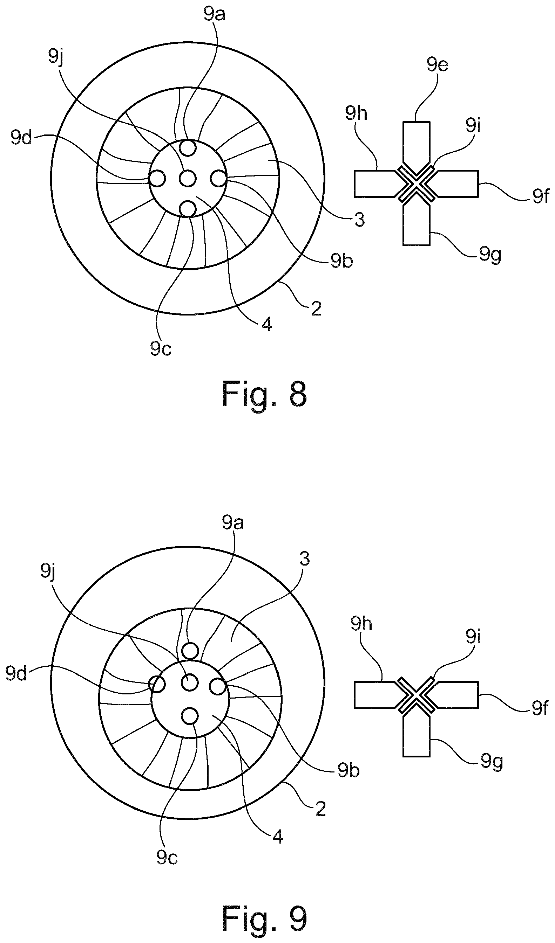

In den Figuren 8 und 9 sind die Bewegung der Pupille 4 und der Iris 3 eines Auges und die Folgen der Sichtbarkeit der verschiedenen Graphikelemente 9e bis 9i detailliert dargestellt.

In der Figur 8 ist das Auge 2 mit der neutralen Blickrichtung geradeaus dargestellt. Die Strahlen 9a, 9b, 9c, 9d, 9j die die Graphikelemente durch die Pupille auf die Netzhaut abbilden, können alle die Pupille passieren. Entsprechend sind alle Graphikelemente9e bis 9i wahrnehmbar, wie dies auf der rechten Seite der Figur dargestellt ist.

In der Figur 9 ist das Auge 2 mit einer leicht nach unten gerichteten Ausrichtung oder Blickrichtung dargestellt. Der Lichtstrahl 9a und das Graphikelement 9e verschwinden in Figur 9 aus dem Blickfeld und die übrigen Graphikelemente 9f, 9g, 9h, 9i bleiben wenigstens teilweise sichtbar, so dass der Blick des Patienten zur Korrektur wieder in Richtung der neutralen Position und des zentralen Graphikelementes 9i gelenkt wird.

Mit diesem Mechanismus, wie insgesamt durch das Konzept der beschriebenen Vorrichtung, können durch die Bildaufnahmeeinrichtung auch bei einer zusammengezogenen Pupille während der Laserbehandlung zufriedenstellende Bilder der Netzhaut zur Überwachung und Steuerung der Laserbehandlung aufgenommen werden. Der Laserstrahl kann ebenfalls geeignet nachgeführt werden, so dass eine Laserbehandlung auch ohne eine Dilation der Pupille mittels Medikamentengabe ermöglicht ist.

## Patentansprüche

1. Vorrichtung zur Behandlung der Netzhaut (1) eines Auges (2) mit einem Behandlungslaser (5), der einen auf die Netzhaut gerichteten und lenkbaren Behandlungslaserstrahl erzeugt,
mit einer Bildaufnahmeeinrichtung (6) zur Aufnahme von Fundusbildern der Netzhaut und mit einer Positioniereinrichtung (8), die die Bildaufnahmeeinrichtung relativ zur Netzhaut positioniert,
**dadurch gekennzeichnet, dass** die Positioniereinrichtung eine Nachführeinrichtung aufweist mit:
einer eye-tracking- Einrichtung (8a), die laufend die Ausrichtung des behandelten Auges relativ zur Bildaufnahmeeinrichtung oder relativ zum Behandlungslaser, insbesondere die Blickrichtung des behandelten Auges ermittelt,
einer ersten Verschiebeeinrichtung (8b) zur Verschiebung der Bildaufnahmeeinrichtung in einer oder mehreren Richtungen quer, insbesondere senkrecht, zur Verbindungslinie (10) (zwischen der Bildaufnahmeeinrichtung und der Netzhaut und
einer Nachführungssteuerung (8e), die die erste Verschiebeeinrichtung in Abhängigkeit von der ermittelten Ausrichtung des behandelten Auges und/oder in Abhängigkeit von der Lage des jeweils zu behandelnden Bereiches auf der Netzhaut steuert.

2. Vorrichtung nach Anspruch 1, **dadurch gekennzeichnet, dass** die erste Verschiebeeinrichtung (8b) dazu eingerichtet ist, gemeinsam mit der Bildaufnahmeeinrichtung (6) eine Quelle (5) oder ein Einkoppelelement (5b) des Behandlungslaserstrahls (5a) und/oder eine Beleuchtungseinrichtung (7) für die Netzhaut (1) und/oder eine Einrichtung zur Temperaturmessung an der Netzhaut zu verschieben.

3. Vorrichtung nach Anspruch 1 oder 2, **dadurch gekennzeichnet, dass** die eye-tracking- Einrichtung (8a) dazu eingerichtet ist, die Ausrichtung des Auges (2), dessen Netzhaut (1) behandelt wird, durch Erfassung der Position und/oder Bewegung von einem oder mehreren Elementen (3,4) des Auges auf seiner der Bildaufnahmeeinrichtung (6) zugewandten Seite zu ermitteln.

4. Vorrichtung nach einem der Ansprüche 1 bis 3, **dadurch gekennzeichnet, dass** die eye-tracking- Einrichtung (8a) eine oder zwei oder mehr als zwei Kameras (11a, 11b) aufweist, die ein oder mehrere Elemente (3,4) auf der der Bildaufnahmeeinrichtung (6) zugewandten Seite des behandelten Auges (2) erfassen.

5. Vorrichtung nach einem der Ansprüche 1 bis 4, **dadurch gekennzeichnet, dass** die Nachführeinrichtung eine zweite Verschiebeeinrichtung (8c) zur Verschiebung der Bildaufnahmeeinrichtung in Richtung der Verbindungslinie (10) zwischen der Bildaufnahmeeinrichtung (6) und der Netzhaut (1) oder der Pupille (4) aufweist.

6. Vorrichtung nach einem der Ansprüche 1 bis 5, **dadurch gekennzeichnet, dass** das mit der Bildaufnahmeeinrichtung (6) erfasste Bild der Netzhaut (1) bei der Erfassung der Ausrichtung des Auges (2) berücksichtigt wird.

7. Vorrichtung nach einem der Ansprüche 1 bis 6, **dadurch gekennzeichnet, dass** die Nachführeinrichtung eine Winkelnachführeinrichtung (8d) aufweist, die bei einer Verschiebung der Bildaufnahmeeinrichtung (6) diese in Abhängigkeit von Richtung und Maß der Verschiebung durch eine Dreh- oder Schwenkbewegung auf die Netzhaut (1) des behandelten Auges (2) ausrichtet.

8. Vorrichtung nach einem der Ansprüche 1 bis 7, **dadurch gekennzeichnet, dass** die Nachführeinrichtung eine Zuordnungseinrichtung (8f) aufweist, die verschiedenen Ausrichtungen des Auges (2) Schwenk- oder Drehrichtungen und/oder Verschiebungsrichtungen der Nachführeinrichtung und Schwenk- oder Drehwinkel und/oder Verschiebungsstrecken zuordnet, um die die Bildaufnahmeeinrichtung (6) durch die Winkelnachführeinrichtung zu schwenken oder drehen und/oder durch eine erste oder zweite Verschiebeeinrichtung (8b, 8c) zu verschieben ist.

9. Vorrichtung nach einem der Ansprüche 1 bis 8, **dadurch gekennzeichnet, dass** die Nachführeinrichtung eine Zuordnungseinrichtung (8f) aufweist, die verschiedenen Ausrichtungen des Auges (2) Zielpositionen der Bildaufnahmeeinrichtung (6) zuordnet, auf die diese ausgerichtet werden soll, oder, die verschiedenen Ausrichtungen des Auges (2) Verschiebungsrichtungen und Verschiebungsstrecken zuordnet, um die die Bildaufnahmeeinrichtung zu verschieben ist.

10. Vorrichtung nach einem der Ansprüche 1 bis 9, **gekennzeichnet durch** eine Leiteinrichtung (9) für die Blickrichtung des Auges (2), dessen Netzhaut (1) behandelt wird, wobei durch die Leiteinrichtung Bilder von wenigstens einem, insbesondere wenigstens zwei, weiter insbesondere fünf Graphikelementen (9e, 9f, 9g, 9h, 9i) mittels separat und im Abstand voneinander durch die Pupille geführter Lichtstrahlen (T, R, B, L, C, 9a, 9b, 9c, 9d, 9j) auf der Netzhaut erzeugt werden und wobei in Abhängigkeit von der Blickrichtung des behandelten Auges (2) von den Lichtstrahlen und den durch diese erzeugten Bildern von Graphikelementen einer/eines oder mehrere durch eine elektronische Steuerung oder optisch durch die Iris (3) des behandelten Auges blockiert werden können.

11. Vorrichtung nach einem der Ansprüche 1 bis 10, **dadurch gekennzeichnet, dass** der Behandlungslaser (5) für eine Behandlung der Netzhaut (1) mit einer Bestrahlungsintensität eingerichtet ist, die das Gewebe der Netzhaut für einen kontrollierbaren Zeitraum auf Temperaturen von weniger als 58 Grad Celsius, insbesondere mit einer Höchsttemperatur zwischen 48 Grad und 58 Grad, weiter insbesondere mit einer Höchsttemperatur zwischen 50 Grad und 55 Grad erwärmt.

12. Verfahren zur Behandlung der Netzhaut (1) eines Auges (2)
mit einem Behandlungslaser (5), der einen auf die Netzhaut (1) gerichteten und lenkbaren Behandlungslaserstrahl (5a) erzeugt, wobei während der Laserbehandlung
mit einer Bildaufnahmeeinrichtung (6) Fundusbilder der Netzhaut aufgenommen werden,
**dadurch gekennzeichnet, dass** während der Laserbehandlung der Netzhaut
mit einer eye-tracking- Einrichtung (8a, 11a, 11b) laufend die Ausrichtung des behandelten Auges relativ zur Bildaufnahmeeinrichtung oder relativ zum Behandlungslaser, insbesondere die Blickrichtung des behandelten Auges ermittelt wird und
mit einer Nachführungssteuerung (8e) in Abhängigkeit von der ermittelten Ausrichtung des behandelten Auges und/oder in Abhängigkeit von der Lage des jeweils zu behandelnden Bereiches auf der Netzhaut eine erste Verschiebeeinrichtung (8b) zur Verschiebung der Bildaufnahmeeinrichtung in einer oder mehreren Richtungen quer, insbesondere senkrecht, zur Verbindungslinie (10) zwischen der Bildaufnahmeeinrichtung und der Netzhaut gesteuert wird.
